(19) Europäisches Patentamt
European Patent Office
Office européen des brevets

(11) Veröffentlichungsnummer: **0 033 083**
**B1**

(12) # EUROPÄISCHE PATENTSCHRIFT

(45) Veröffentlichungstag der Patentschrift: **08.08.84**

(51) Int. Cl.³: **C 07 D 231/28**, A 01 N 47/18

(21) Anmeldenummer: **81100188.2**

(22) Anmeldetag: **13.01.81**

(54) **N,N-Dialkyl -0-(pyrazol-5-yl)-carbaminsäureester, Verfahren zu ihrer Herstellung und ihre Verwendung als Insektizide.**

(30) Priorität: **24.01.80 DE 3002413**

(43) Veröffentlichungstag der Anmeldung: **05.08.81 Patentblatt 81/31**

(45) Bekanntmachung des Hinweises auf die Patenterteilung: **08.08.84 Patentblatt 84/32**

(84) Benannte Vertragsstaaten: **AT BE CH DE FR GB IT LI NL SE**

(56) Entgegenhaltungen:
**DE - A - 2 644 588**
**DE - A - 2 644 589**
**DE - A - 2 721 188**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Stähler, Gerhard, Dr., Loreleistrasse 87, D-6230 Frankfurt am Main 80 (DE)**
Erfinder: **Waltersdorfer, Anna, Dr., Rauenthaler Weg 28, D-6000 Frankfurt am Main 71 (DE)**

**EP 0 033 083 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue N,N-Dialkyl-O-[pyrazol-5-yl]-carbaminsäureester der allgemeinen Formel I

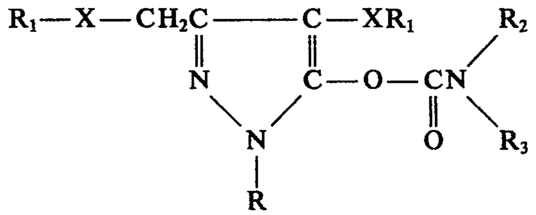

(I)

in welcher
R ($C_1-C_8$)-Alkyl, das durch CN substituiert sein kann,
$R_1$, $R_2$ und $R_3$ ($C_1-C_4$)-Alkyl und
X Sauerstoff oder Schwefel bedeuten. Die erfindungsgemäßen Verbindungen lassen sich herstellen, indem man Pyrazolone der allgemeinen Formel II

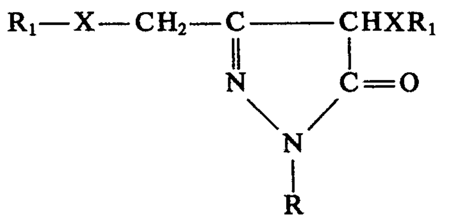

(II)

worin R, $R_1$ die unter Formel I angegebenen Bedeutungen haben, in Form ihrer Alkali-, Erdalkali- oder Ammoniumsalze oder in Gegenwart von Säureakzeptoren mit N,N-Dialkylcarbaminsäurehalogeniden der Formel III

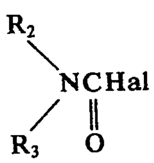

(III)

umsetzt.

Die als Ausgangsstoff III verwendeten N,N-Dialkylcarbaminsäurechloride sind bekannt und können nach bekannten Verfahren hergestellt werden. Die Pyrazolone der Formel II können durch Umsetzung von substituierten Hydrazinen der Formel (IV)

$RNHNH_2$ (IV)

mit Acetessigesterderivaten der Formel (V)

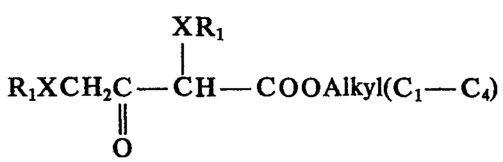

(V)

worin R, $R_1$ und X die unter Formel I angegebenen Bedeutungen haben, erhalten werden. Letztere sind in literaturbekannter Weise durch eine Acetessigesterkondensation nach Claisen aus 2 Mol Alkoxy- bzw. Alkylmercapto-acetessigester erhältlich.

Die Umsetzung zu den erfindungsgemäßen Verbindungen wird vorzugsweise in inerten Lösungs- oder Verdünnungsmitteln vorgenommen. Hierzu eignen sich beispielsweise aliphatische und aromatische, gegebenenfalls chlorierte Kohlenwasserstoffe wie Benzol, Toluol, Xylol, Chlorbenzol, Methylenchlorid, Chloroform, Tetrachlorkohlenstoff, 1,2-Dichlorethan oder Ether wie Diethyl-, Dipropyl-, Dibutyl- oder Glykoldimethylether, des weiteren Ketone wie Aceton oder Methylethylketon oder Nitrile wie Acetonitril oder Propionitril.

Die verwendeten Pyrazolone können in Form der Alkalisalze ihrer tautomeren Form oder in Gegenwart eines Säureakzeptors mit dem N,N-Dialkylcarbaminsäurechloriden umgesetzt werden. Als Säurebindemittel eignen sich besonders Alkalicarbonate oder -alkoholate wie Natrium- oder Kaliumcarbonat oder Kalium-tert.-Butylat oder organische Basen wie zum Beispiel Triethylamin, Dimethylanilin und Pyridin.

Die Reaktion findet bei Normaldruck im allgemeinen zwischen Raumtemperatur und 100° C, vorzugsweise zwischen 30 und 70° C statt.

Die Ausgangsstoffe werden im allgemeinen in stöchiometrischen Mengen oder mit einem geringen Überschuß an N,N-Dialkylcarbaminsäurechlorid eingesetzt.

Die Reaktionsprodukte fallen im allgemeinen in Form nicht destillierbarer Öle, seltener in fester Form mit niedrigem Schmelzpunkt an.

Die erfindungsgemäßen Verbindungen I zeichnen sich durch hervorragende selektive insektizide und akarizide Wirksamkeit, insbesondere gegen Aphidina (Blattläuse) aus. Besonders hervorzuheben ist dabei ihre systematische Wirksamkeit. Das heißt, daß sie sowohl nach Aufnahme über grüne Pflanzenteile als auch nach Aufnahme über das Wurzelsystem wirken. Es ist daher mit ihrer Hilfe möglich, versteckt lebende Blattlausarten innerhalb von Pflanzengallen und anderen nicht direkt erreichbaren Pflanzenteilen gut zu bekämpfen.

Zu den mit den erfindungsgemäßen Verbindungen bekämpfbaren Blattlausarten gehören die Grüne Pfirsichblattlaus (Myzus persicae), die Schwarze Bohnenblattlaus (Aphis fabae), die Haferblattlaus (Rhopalosiphum padi), die Grüne Erbsenblattlaus (Acyrtosiphon pisum), die Mehlige Kohlblattlaus (Brevicoryne brassicae), ferner die Johannisbeerblasenlaus (Cryptomycus ribis), die Mehlige Apfellaus (Dysaphis radicicola), die Schwarze Kirschenblattlaus (Mycus cerasi) und die Blutlaus (Eriosoma lanigerum).

Die erfindungsgemäßen Verbindungen können außerdem zur Bekämpfung einer großen Zahl von tierischen Schädlingen, wie z. B. aus den Ordnungen der Orthoptera, Heteroptera, Homoptera, Lepidoptera, Hymenoptera, Diptera, Coleoptera und Acarina, die in der Landwirtschaft, im Forst und im Vorrats- und Hygienesektor vorkommen, eingesetzt werden.

Gegenstand der Erfindung sind daher auch Schädlingsbekämpfungsmittel, die Verbindungen der allgemeinen Formel I als Wirkstoffe enthalten.

Die erfindungsgemäßen Mittel enthalten die Wirkstoffe der Formel I im allgemeinen zu 2—80 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Fettalkohole, Alkyl- oder Alkylphenyl-sulfonate und Dispergiermittel, z. B. ligninsulfonsaures Natrium, 2,2'-Dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden:

alkylarylsulfonsaure Calziumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxyethylen-sorbitan-fettsäureester oder Polyoxethylensorbitester.

Stäubemittel erhält man durch Vermahlen des Wirkstoffes mit fein verteilten, festen Stoffen, z. B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen auf die Oberfläche von Trägerstoffen, wie Sand, Kaolinite, oder von granuliertem Inertmaterial. Auch können geeignete Wirkstoffe in der für die Herstellung von Düngemittelgranalien üblichen Weise — gewünschtenfalls in Mischung mit Düngemitteln — hergestellt werden.

In Spritzpulvern variiert die Wirkstoffkonzentration z. B. zwischen etwa 10% und 80%, der Rest besteht aus den oben angegebenen Formulierungszusätzen. Bei emulgierbaren Konzentraten kann die Wirkstoffkonzentration gleichfalls etwa 10% bis 80% betragen. Staubförmige Formulierungen enthalten meistens 5 bis 20% an Wirkstoff, versprühbare Lösungen etwa 2 bis 20%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Die erfindungsgemäßen Wirkstoffe können mit Fungiziden oder anderen Insektiziden und Akariziden kombiniert werden.

Die Erfindung wird durch folgende Beispiele näher erläutert:

A) Herstellungsbeispiele

Beispiel 1

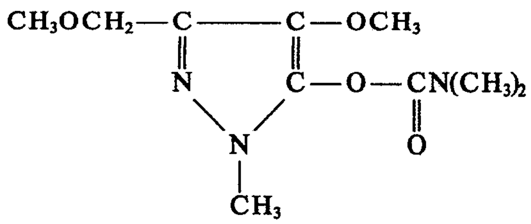

17 g 1-Methyl-3-methoxymethyl-4-methoxypyrazolon-5 werden in 150 ml absolutem Acetonitril gelöst. Nach Zusatz von 12 g Kalium-tert.-Butylat wird das Reaktionsgemisch homogen gerührt und nach Zusatz von 11 g Dimethylcarbaminsäurechlorid 2 Stunden auf 50—60°C erhitzt. Dann wird das Lösungsmittel im Rotationsverdampfer bei 100—200 Torr abdestilliert. Der Rückstand wird in 150 ml Methylenchlorid aufgenommen und zweimal mit je 30 ml Wasser ausgeschüttet. Die abgeschiedene Methylenchloridphase wird nach Trocknen mit wasserfreiem Natriumsulfat in gelindem Vacuum eingedunstet. Zurück bleiben 23 g 1-Methyl-3-methoxymethyl-4-methoxy-5-dimethylcarbamoylpyrazol (94,7% d. Th.) als hellbraunes Öl mit dem Brechungsindex $n_D^{23} = 1,4970$.

In ähnlicher Weise wurden folgende Verbindungen der allgemeinen Formel

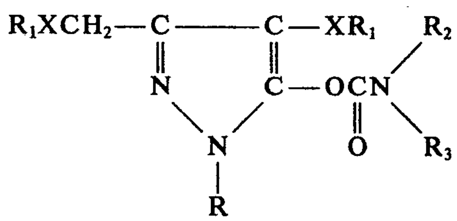

hergestellt:

| Beispiel Nr. | R | $R_1$ | $R_2$ | $R_3$ | X | Physikalische Daten |
|---|---|---|---|---|---|---|
| 2 | $-CH_3$ | $-CH_3$ | $-CH_3$ | $-CH_3$ | S | Fp. 87–89°C |
| 3 | $-CH_3$ | $-C_2H_5$ | $-CH_3$ | $-CH_3$ | O | $n_D^{23}$ = 1,4849 |
| 4 | $-CH_3$ | $-C_2H_5$ | $-CH_3$ | $-CH_3$ | S | $n_D^{23}$ = 1,5360 |
| 5 | $-C_2H_5$ | $-CH_3$ | $-CH_3$ | $-CH_3$ | O | $n_D^{23}$ = 1,4922 |
| 6 | $-C_2H_5$ | $-CH_3$ | $-CH_3$ | $-CH_3$ | S | Fp. 56–58°C |
| 7 | $-C_2H_5$ | $-C_2H_5$ | $-CH_3$ | $-CH_3$ | S | $n_D^{23}$ = 1,5217 |
| 8 | $-CH_2CH_2CN$ | $-CH_3$ | $-CH_3$ | $-CH_3$ | O | $n_D^{23}$ = 1,4997 |
| 9 | $-CH_2CH_2CN$ | $-CH_3$ | $-CH_3$ | $-CH_3$ | S | $n_D^{23}$ = 1,5270 |
| 10 | $-C_3H_{7\,(n)}$ | $-CH_3$ | $-CH_3$ | $-CH_3$ | O | $n_D^{23}$ = 1,4831 |
| 11 | $-C_3H_{7\,(n)}$ | $-CH_3$ | $-CH_3$ | $-CH_3$ | S | Fp. 55–56°C |
| 12 | $-C_3H_{7\,(n)}$ | $-C_2H_5$ | $-CH_3$ | $-CH_3$ | O | $n_D^{23}$ = 1,4780 |
| 13 | $-C_3H_7$ (i) | $-CH_3$ | $-CH_3$ | $-CH_3$ | O | $n_D^{23}$ = 1,4872 |
| 14 | $-C_3H_7$ (i) | $-CH_3$ | $-CH_3$ | $-CH_3$ | S | Fp. 68–70°C |
| 15 | $-C_3H_7$ (i) | $-C_2H_5$ | $-CH_3$ | $-CH_3$ | S | $n_D^{23}$ = 1,5231 |
| 16 | $-C_4H_9$ (n) | $-CH_3$ | $-CH_3$ | $-CH_3$ | O | $n_D^{23}$ = 1,4782 |
| 17 | $-C_4H_9$ (n) | $-CH_3$ | $-CH_3$ | $-CH_3$ | S | $n_D^{23}$ = 1,5362 |
| 18 | $-C_4H_9$ (n) | $-C_2H_5$ | $-CH_3$ | $-CH_3$ | O | $n_D^{23}$ = 1,4796 |
| 19 | $-CH(CH_3)CH_2CH_3$ | $-CH_3$ | $-CH_3$ | $-CH_3$ | O | $n_D^{23}$ = 1,4897 |
| 20 | $-CH(CH_3)CH_2CH_3$ | $-CH_3$ | $-CH_3$ | $-CH_3$ | S | Fp. 59–61°C |
| 21 | $-C_4H_9$ (i) | $-CH_3$ | $-CH_3$ | $-CH_3$ | O | $n_D^{23}$ = 1,4851 |
| 22 | $-C_4H_9$ (i) | $-CH_3$ | $-CH_3$ | $-CH_3$ | S | Fp. 49–51°C |
| 23 | $-C_5H_{11}$ (n) | $-CH_3$ | $-CH_3$ | $-CH_3$ | O | $n_D^{23}$ = 1,4726 |
| 24 | $-C_5H_{11}$ (n) | $-CH_3$ | $-CH_3$ | $-CH_3$ | S | $n_D^{23}$ = 1,5321 |
| 25 | $-C_2H_5$ | $-CH_3$ | $-C_2H_5$ | $-C_2H_5$ | O | $n_D^{20}$ = 1,4848 |
| 26 | $-C_4H_9$ (i) | $-CH_3$ | $-C_2H_5$ | $-C_2H_5$ | O | $n_D^{20}$ = 1,4771 |
| 27 | $-CH_2CH_2CN$ | $-CH_3$ | $-C_2H_5$ | $-C_2H_5$ | O | $n_D^{20}$ = 1,4929 |
| 28 | $-C_5H_{11}$ (n) | $-CH_3$ | $-C_2H_5$ | $-C_2H_5$ | O | $n_D^{20}$ = 1,4795 |
| 29 | $-C_3H_7$ (i) | $-CH_3$ | $-C_3H_7$ (n) | $-C_3H_7$ (n) | S | $n_D^{20}$ = 1,5230 |

## B) Formulierungsbeispiele

### Beispiel a

Ein emulgierbares Konzentrat wird erhalten aus

| | |
|---|---|
| 15 Gew.-Teilen | Wirkstoff |
| 75 Gew.-Teilen | Cyclohexanon als Lösungsmittel |
| und 10 Gew.-Teilen | oxethyliertem Nonylphenol (10 AeO) als Emulgator. |

### Beispiel b

Ein in Wasser leicht dispergierbares benetzbares Pulver wird erhalten, indem man

| | |
|---|---|
| 25 Gew.-Teile | Wirkstoff |
| 64 Gew.-Teile | aktive Kieselsäure als Inertstoff |
| 10 Gew.-Teile | ligninsulfonsaures Calcium |
| und 1 Gew.-Teil | oleylmethyltaurinsaures Natrium als Netz- und Dispergiermittel |

mischt und in einer Stiftmühle mahlt.

### Beispiel c

Ein Stäubemittel wird erhalten, indem man

| | |
|---|---|
| 10 Gew.-Teile | Wirkstoff |
| und 90 Gew.-Teile | Talkum als Inertstoff |

mischt und in einer Schlagmühle zerkleinert.

### Beispiel d

Ein Granulat besteht z. B. aus etwa

| | |
|---|---|
| 2—15 Gew.-Teilen | Wirkstoff |
| 98—85 Gew.-Teilen | inerten Granulatmaterialien, wie z. B. Attapulgit, Bimsstein und Quarzsand. |

## C) Biologische Beispiele

### Beispiel I

Mit Kundebohnenblattläusen (Aphis craccivora) stark besetzte Ackerbohnen (Vicia faba) wurden mit der wäßrigen Suspension eines Spritzpulverkonzentrates, die 0,025 Gew.-% des Wirkstoffs aus Beispiel 1 enthielt, bis zum Stadium des Abtropfens gespritzt.

Nach Aufstellung der Pflanzen im Gewächshaus wurde 3 Tage nach der Behandlung eine 100%ige Abtötung der Versuchstiere festgestellt. Ebenso wirksam erwiesen sich die Verbindungen gemäß Beispielen 2—8, 10—24. Die Verbindungen der Beispiele 9, 25—29 waren in einer Konzentration von 0,2% 3 Tage nach der Behandlung 100%ig wirksam.

### Beispiel II

Getopfte Ackerbohnen (Vicia faba), deren Wurzelballen mit Folie eingehüllt sind, werden nach erfolgter Infektion mit Kundebohnenblattläusen (Aphis craccivora) mit 1 mg Wirkstoff in der Weise behandelt, daß mittels eines Glastrichters eine wäßrige Verdünnung eines Emulsionskonzentrates im Wurzelbereich gleichmäßig verteilt wird.

Die nach 8 Tagen durchgeführte Auswertung ergab für die Verbindungen gemäß Beispielen 1, 3, 5, 6, 8, 10—21 und 23 100% Mortalität.

**Patentansprüche für die Vertragsstaaten: BE, CH, DE, FR, GB, IT, LI, NL, SE**

1. N,N-Dialkyl-O-[pyrazol-5-yl]-carbaminsäureester der allgemeinen Formel I

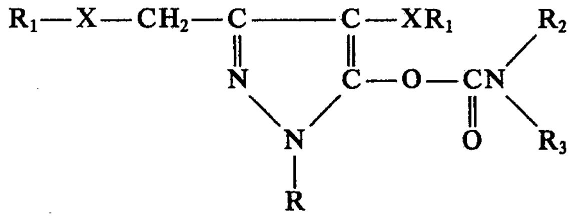

(I)

in welcher
R $(C_1-C_8)$-Alkyl, das durch CN substituiert sein kann,
$R_1$, $R_2$ und $R_3 = (C_1-C_4)$-Alkyl und
X Sauerstoff oder Schwefel bedeuten.

2. Verfahren zur Herstellung von Verbindungen der Formel I, dadurch gekennzeichnet, daß man Pyrazolone der allgemeinen Formel II

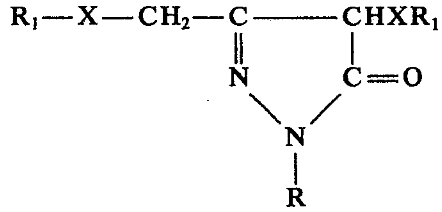

(II)

worin R, $R_1$ die unter Formel I angegebenen Bedeutungen haben, in Form ihrer Alkali-, Erdalkali- oder Ammoniumsalze oder in Gegenwart von Säureakzeptoren mit N,N-Dialkylcarbaminsäurehalogeniden der Formel III

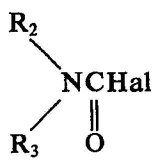

(III)

umsetzt.

3. Insektizide und akarizide Mittel, gekennzeichnet durch einen Gehalt an einer Verbindung der Formel I.

4. Verwendung von Verbindungen der Formel I zur Bekämpfung von Insekten und Akariden.

5. Verfahren zur Bekämpfung von schädlichen Insekten und Akariden, dadurch gekennzeichnet, daß man auf die befallenen Nutzpflanzen eine wirksame Menge einer Verbindung der Formel I aufbringt.

6. 1-Cyanäthyl-3-methoxymethyl-4-methoxy-5-dimethylcarbamoyloxypyrazol.

7. 1-n-Propyl-3-methoxymethyl-4-methoxy-5-dimethylcarbamoyloxypyrazol.

8. 1-n-Butyl-3-methoxymethyl-4-methoxy-5-dimethylcarbamoyloxypyrazol.

9. 1-n-Amyl-3-methoxymethyl-4-methoxy-5-dimethylcarbamoyloxypyrazol.

**Patentansprüche für den Vertragsstaat: AT**

1. Verfahren zur Herstellung von Verbindungen der Formel I

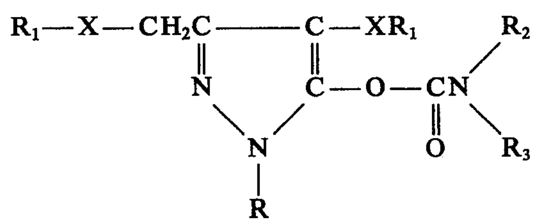

(I)

in welcher
R $(C_1-C_8)$-Alkyl, das durch CN substituiert sein kann,
$R_1$, $R_2$ und $R_3$ $(C_1-C_4)$-Alkyl und

X Sauerstoff oder Schwefel bedeuten, dadurch gekennzeichnet, daß man Pyrazolone der allgemeinen Formel II

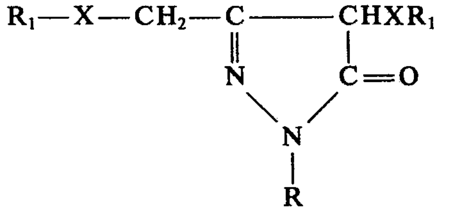

(II)

worin R, $R_1$ die unter Formel I angegebenen Bedeutungen haben, in Form ihrer Alkali-, Erdalkali- oder Ammoniumsalze oder in Gegenwart von Säureakzeptoren mit N,N-Dialkylcarbaminsäurehalogeniden der Formel III

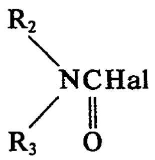

(III)

umsetzt.

2. Insektizide und akarizide Mittel, gekennzeichnet durch einen Gehalt an einer Verbindung der Formel I.

3. Verwendung von Verbindungen der Formel I zur Bekämpfung von Insekten und Akariden.

4. Verfahren zur Bekämpfung von schädlichen Insekten und Akariden, dadurch gekennzeichnet, daß man auf die befallenen Nutzpflanzen eine wirksame Menge einer Verbindung der Formel I aufbringt.

**Claims for the Contracting states: BE, CH, DE, FR, GB, IT, LI, NL, SE**

1. N,N-dialkyl-O-[pyrazol-5-yl]-carbamic acid esters of the general formula I

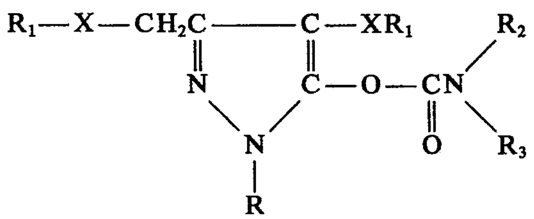

(I)

wherein

R is $C_1-C_8$ alkyl optionally substituted with CN,

$R_1$, $R_2$ and $R_3$ are $C_1-C_4$ alkyl and

X is oxygen or sulfur.

2. Process for the manufacture of compounds of the formula I which comprises reacting pyrazolones of the general formula II

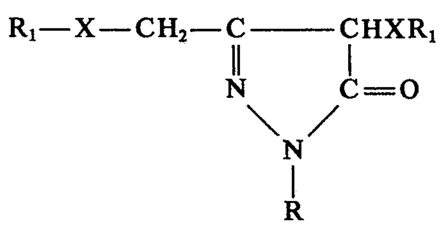

(II)

in which R and $R_1$ are as defined under formula I, in the presence of acid acceptors or in the form of their alkali metal alkaline earth metal or ammonium salts, with N,N-dialkylcarbamic acid halides of the formula III

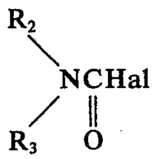

(III)

3. Insecticidal and acaricidal composition containing a compound of the formula I.
4. The use of the compounds of formula I for combating insects and acaridae.
5. Method of combating noxious insects and acaridae which comprises treating the infested crop plants with an effective amount of a compound of the formula I.
6. 1-Cyanoethyl-3-methoxymethyl-4-methoxy-5-dimethylcarbamoyloxypyrazole.
7. 1-n-Propyl-3-methoxymethyl-4-methoxy-5-dimethylcarbamoyloxypyrazole.
8. 1-n-Butyl-3-methoxymethyl-4-methoxy-5-dimethylcarbamoyloxypyrazole.
9. 1-n-Amyl-3-methoxymethyl-4-methoxy-5-dimethylcarbamoyloxypyrazole.

**Claims for the Contracting state: AT**

1. Process for the manufacture of compounds of the formula I

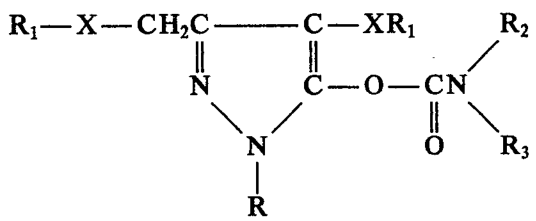

(I)

wherein
R is $C_1-C_8$ alkyl optionally substituted with CN,
$R_1$, $R_2$ and $R_3$ are $C_1-C_4$ alkyl and
X is oxygen or sulfur which comprises reacting pyrazolones of the general formula II

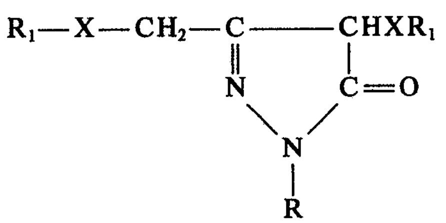

(II)

in which R and $R_1$ are as defined under formula I, in the presence of acid acceptors or in the form of their alkali metal, alkaline earth metal or ammonium salts, with N,N-dialkylcarbamic acid halides of the formula III

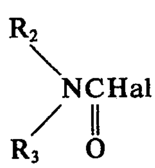

(III)

2. Insecticidal and acaricidal composition containing a compound of the formula I.
3. The use of the compounds of formula I for combating insects and acaridae.
4. Method of combating noxious insects and acaridae which comprises treating the infested crop plants with an effective amount of a compound of the formula I.

**Revendications pour les Etats contractants: BE, CH, DE, FR, GB, IT, LI, NL, SE**

1. N,N-Dialkyl-carbamates de pyrazolyles-5 répondant à la formule générale I:

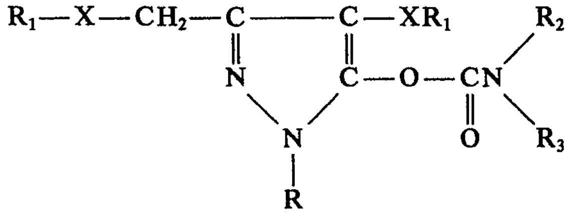

(I)

dans laquelle
R représente un radical alkyle en $C_1-C_8$ qui peut porter un radical CN,
$R_1$, $R_2$ et $R_3$ représentent chacun un radical alkyle en $C_1-C_4$ et
X représente l'oxygène ou le soufre.

2. Procédé de préparation de composés de formule I, procédé caractérisé en ce que l'on fait réagir des pyrazolones répondant à la formule générale II:

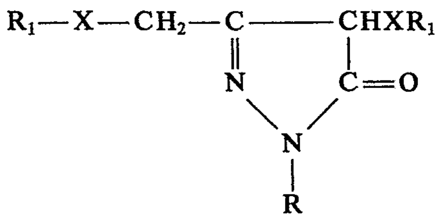

(II)

dans laquelle R et $R_1$ ont les significations données à propos de la formule I, en les utilisant sous la forme de leurs sels de métaux alcalins, de métaux alcalinoterreux ou d'ammonium ou en présence d'accepteurs d'acides, avec des halogénures de N,N-dialkyl-carbamoyles de formule III:

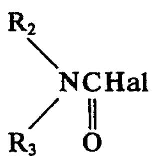

(III)

3. Produits insecticides et acaricides caractérisés en ce qu'ils contiennent un composé de formule I.
4. Application de composés de formule I à la lutte contre des insectes et des acarides.
5. Procédé pour combattre des insectes et des acarides nuisibles, procédé caractérisé en ce qu'on applique, sur les plantes utiles attaquées, une quantité efficace d'un composé de formule I.
6. Cyanéthyl-1 méthoxyméthyl-3 méthoxy-4 diméthylcarbamoyloxy-5 pyrazole.
7. n-Propyl-1 méthoxyméthyl-3 méthoxy-4 diméthylcarbamoyloxy-5 pyrazole.
8. n-Butyl-1 méthoxyméthyl-3 méthoxy-4 diméthylcarbamoyloxy-5 pyrazole.
9. n-Pentyl-1 méthoxyméthyl-3 méthoxy-4 diméthylcarbamoyloxy-5 pyrazole.

**Revendications pour l'Etat contractant: AT**

1. Procédé de préparation de composés répondant à la formule I:

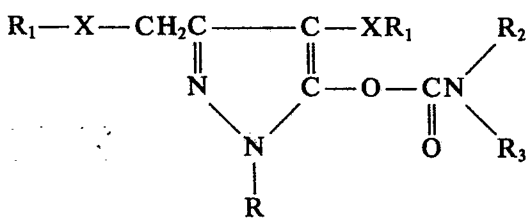

(I)

dans laquelle
R représente un radical alkyle en $C_1-C_8$ qui peut porter un radical CN,
$R_1$, $R_2$ et $R_3$ représentent chacun un radical alkyle en $C_1-C_4$ et
X représente l'oxygène ou le soufre, procédé caractérisé en ce qu'on fait réagir des pyrazolones répondant à la formule générale II:

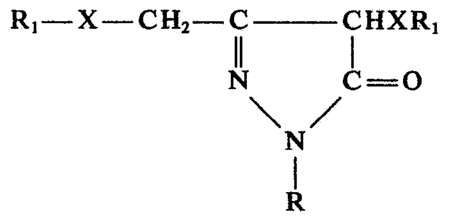

(II)

dans laquelle R et $R_1$ ont les significations indiquées ci-dessus, mises en jeu sous la forme de leurs sels de métaux alcalins, de métaux alcalinoterreux ou d'ammonium ou en présence d'accepteurs d'acides, avec des halogénures de N,N-dialkyl-carbamoyles de formule III:

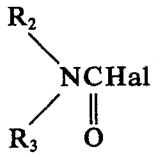

(III)

2. Produits insecticides et acaricides en ce qu'ils contiennent un composé de formule I.
3. Application de composés de formule I à la lutte contre des insectes et des acarides.
4. Procédé pour combattre des insectes et des acarides nuisibles, procédé caractérisé en ce qu'on applique, sur les plantes utiles attaquées, un composé efficace d'un composé de formule I.